# EUROPEAN PATENT APPLICATION

(11) **EP 3 386 090 A1**
(43) Date of publication of application: **10.10.2018**
(21) Application number: 17165250.6
(22) Date of filing: 06.04.2017
(51) Int. Cl.: H02N 1/00, B82B 1/00

(54) **MOLECULAR MACHINE**

(71) Applicant: Technische Universität München, 80333 München (DE)
(72) Inventor: KOPPERGER, Enzo, 81667 München (DE); LIST, Jonathan, 1700 Fribourg (CH); SIMMEL, Friedrich C., 80336 München (DE)
(74) Representative: Hoefer & Partner Patentanwälte mbB

(57) **Abstract**

The invention regards a molecular machine comprising a movement part (2) including a first molecular element (4), a second molecular element (5), and a linking element (6) for constraining a relative movement of the first molecular element (4) and the second molecular element (5), and a control part configured to generate an electrical field around the movement part (2), wherein the first molecular element (4) is fixed relative to the control part, wherein the second molecular element (5) is movable relative to the first molecular element (4) in at least one degree of freedom, and wherein the second molecular element (5) is electrically charged such that the second molecular element (5) aligns to said electrical field.

## Description

The invention regards the field of nanotechnology and describes the utilization of electric fields for the manipulation of molecular mechanisms. In this way, a molecular machine is provided, which allows movement in response to said electrical fields.

### Prior art references:

1. Hess, H.; Bachand, G. D.; Vogel, V., Powering Nanodevices with Biomolecular Motors. Chemistry 2004, 10, 2110-6.
2. van den Heuvel, M. G. L.; Dekker, C., Motor Proteins at Work for Nanotechnology. Science (New York, NY) 2007, 317, 333-336.
3. Fischer, T.; Agarwal, A.; Hess, H., A Smart Dust Biosensor Powered by Kinesin Motors. Nature Nanotech. 2009, 4, 162-166.
4. van den Heuvel, M. G.; de Graaff, M. P.; Dekker, C., Molecular Sorting by Electrical Steering of Microtubules in Kinesin-Coated Channels. Science (New York, NY) 2006, 312, 910-4.
5. Krishnan, Y.; Simmel, F. C., Nucleic Acid Based Molecular Devices. Angew. Chem. Int. Ed. 2011, 50, 3124-3156.
6. Kopperger, E.; Pirzer, T.; Simmel, F. C., Diffusive Transport of Molecular Cargo Tethered to a DNA Origami Platform. Nano Lett. 2015, 15, 2693-2699.
7. Marras, A. E.; Zhou, L.; Su, H.-J.; Castro, C. E., Programmable Motion of DNA Origami Mechanisms. Proc. Natl. Ac. Sci. 2015, 112, 713-8.
8. Ketterer, P.; Willner, E. M.; Dietz, H., Nanoscale Rotary Apparatus Formed from Tight-Fitting 3d DNA Components. Sci Adv 2016, 2, e1501209.
9. List, J.; Falgenhauer, E.; Kopperger, E.; Pardatscher, G.; Simmel, F. C., Long-Range Movement of Large Mechanically Interlocked DNA Nanostructures. Nat Commun 2016, 7, 12414.
10. Omabegho, T.; Sha, R.; Seeman, N. C., A Bipedal DNA Brownian Motor with Coordinated Legs. Science 2009, 324, 67-71.
11. Green, S.; Bath, J.; Turberfield, A., Coordinated Chemomechanical Cycles: A Mechanism for Autonomous Molecular Motion. Phys. Rev. Lett. 2008, 101, art. no. 238101.
12. Liber, M.; Tomov, T. E.; Tsukanov, R.; Berger, Y.; Nir, E., A Bipedal DNA Motor That Travels Back and Forth between Two DNA Origami Tiles. Small 2014, n/a-n/a.
13. Wickham, S. F. J.; Endo, M.; Katsuda, Y.; Hidaka, K.; Bath, J.; Sugiyama, H.; Turberfield, A. J., Direct Observation of Stepwise Movement of a Synthetic Molecular Transporter. Nature Nanotech. 2011, 6, 166-169.
14. Asanuma, H.; Liang, X.; Yoshida, T.; Komiyama, M., Photocontrol of DNA Duplex Formation by Using Azobenzene-Bearing Oligonucleotides. Chembiochem 2001, 2, 39-44.
15. Kang, H.; Liu, H.; Phillips, J. A.; Cao, Z.; Kim, Y.; Chen, Y.; Yang, Z.; Li, J.; Tan, W., Single-DNA Molecule Nanomotor Regulated by Photons. Nano Lett. 2009, 9, 2690-2696.
16. Suzuki, Y.; Endo, M.; Yang, Y.; Sugiyama, H., Dynamic Assembly/Disassembly Processes of Photoresponsive DNA Origami Nanostructures Directly Visualized on a Lipid Membrane Surface. J. Am. Chem. Soc. 2014, 136, 1714-1717.
17. Rothemund, P. W. K., Folding DNA to Create Nanoscale Shapes and Patterns. Nature 2006, 440, 297-302.
18. Douglas, S. M.; Dietz, H.; Liedl, T.; Högberg, B.; Graf, F.; Shih, W. M., Self-Assembly of DNA into Nanoscale Three-Dimensional Shapes. Nature 2009, 459, 414-8.
19. Lin, C.; Jungmann, R.; Leifer, A. M.; Li, C.; Levner, D.; Church, G. M.; Shih, W. M.; Yin, P., Submicrometre Geometrically Encoded Fluorescent Barcodes Self-Assembled from DNA. Nature Chem. 2012, 4, 832-839.
20. Li, X.; Liu, D. R., DNA-Templated Organic Synthesis: Nature's Strategy for Controlling Chemical Reactivity Applied to Synthetic Molecules. Angew. Chem. Int. Ed. 2004, 43, 4848-4870.
21. He, Y.; Liu, D. R., Autonomous Multistep Organic Synthesis in a Single Isothermal Solution Mediated by a DNA Walker. Nature Nanotech. 2010, 5, 778-782.
22. Rant, U.; Pringsheim, E.; Kaiser, W.; Arinaga, K.; Knezevic, J.; Tornow, M.; Fujita, S.; Yokoyama, N.; Abstreiter, G., Detection and Size Analysis of Proteins with Switchable DNA Layers. Nano Letters 2009, Vol. 9, No. 4, 1290-1295.
23. Yang, Y.; Tashiro, R.; Suzuki, Y.; Emura, T.; Hidaka, K.; Sugijama, H.; Endo, M., A Photoregulated DNA-Based Rotary System and Direct Observation of Its Rotational Movement. Chemistry, 2007, 23, 1-8.
24. Campos, R.; Zhang, S.; Majikes, J. M.; Ferraz, L. C. C.; LaBean, T.H.; Dong, M. D.; Ferapotova, E. E., Electronically addressable nanomechanical switching of i-motif DNA origami assembled on basal plane HOPG. ChemComm, 2015, 51, 14111-14114.

In the past decades, several biomolecular mechanisms and machines have been demonstrated for the application in nanotechnology. However, none of them grew past the "proof of principle" phase.

Among the naturally occurring molecular machines mainly kinesin motors and microtubules were deployed, e.g., for the transport of nanoparticles (cf. prior art references 1 to 3). In so-called "in vitro motility assays" kinesin motors were fixed on lithographically patterned chip surfaces to transport microtubules through lithographically produced channel systems. Here electric manipulation was also used e.g. for the sorting of microtubules (cf. prior art reference 4).

In the past years, artificial biomolecular nanomachines were mainly produced on the basis of DNA molecules. The production of DNA machines utilizes the characteristic, sequence specific molecular interactions between DNA strands. Adequate choice of DNA sequences allows to "programmably" construct complex molecular structures from single DNA strands. A combination of relatively flexible single stranded DNA components with more rigid double stranded elements allows the construction of molecular mechanisms in which molecular components can be moved relative to each other. These include "DNA tweezers" as well as various "DNA walker" systems. An overview of such systems is given in prior art reference 5. With the recent development of the so-called "DNA origami method" it has become much easier to construct larger molecular systems (typically on the length scale of 10 - 100 nm). Recently this method was used to construct basic molecular machine elements, including several rotary joint and linear sliding structures (prior art references 6 to 9).

So far, the described molecular mechanisms have been actuated chemically or in some cases photo-chemically. In the case of chemical actuation, e.g., so-called DNA fuel strands were used to drive the movement of a mechanism through DNA strand hybridization reactions. Due to the slow kinetics of these reactions only very slow motion could be realized (prior art references 10 to 12). The same limitations apply to deoxyribozymes and DNA modifying enzymes (prior art reference 13).

Alternatively, motion of molecular mechanisms was achieved with a change of buffer conditions, e.g. by a change in pH or a change of the solution's ionic conditions. These methods come with the disadvantage that they unspecifically influence all system components and that buffer conditions are often not compatible with the chemistry of potential applications (e.g. enzymes are not functional in the specific conditions, nanoparticles can aggregate, etc.)

The aforementioned chemical methods typically require the external addition of solutions. In principle, this can be done with the help of microfluidic systems, which could allow a certain degree of automation. However, such an approach requires rather elaborate instruments and causes high material consumption.

At first glance, an attractive method for external control of nanomachines is based on photoswitchable molecules, typically derivates of the photoswitchable molecule azobenzene (prior art references 14 to 16). Incorporation of such photoswitches into DNA double strands makes it possible to destabilize them upon light irradiation (in UV range) and stabilize them by irradiation with light of larger wavelength, and this procedure can be used to drive molecular mechanisms. The downside of this method, apart from the necessary chemical modification of the mechanisms, again is the slow and incomplete switching behavior.

Prior art reference 22 describes a biosensor including a gold platform. This biosensor allows only binary switching between an adhesive state and a non-adhesive state. No fine adjustment of the movement is possible. Prior art reference 23 describes the movement of molecular mechanisms in response to UV light.

None of the described approaches could ever demonstrate the exertion of a relevant force against an external load.

It is an object of the present invention to provide a molecular machine which allows quick reaction to control commands, which can generate high forces, and which can provide an exact movement.

The object is solved by the features of the independent claim. The dependent claims contain advantageous embodiments of the present invention.

The object is therefore solved by a molecular machine comprising a movement part and a control part. The movement part comprises several machine elements which are adapted to be moved with respect to each other. These machine parts are molecular structures, particularly nanomolecular structures. The movement part includes a first molecular element, a second molecular element and a linking element. The first molecular element and the second molecular element preferably are separate and/or independent elements. The linking element is adapted to constrain a relative movement of the first molecular element and the second molecular element while allowing a relative movement between the first molecular element and the second molecular element in at least one degree of freedom. Preferably, the first molecular element and the second molecular element can not be separated from each other and establish a moving mechanism with the linking element acting as bearing and/or joint.

In order to control the relative movement of first molecular element and second molecular element, the control part is configured to generate an electrical field around the movement part. Preferably, the control part includes an electrical device which applies said electrical field. The first molecular element is fixed relative to the control part such that the first molecular element is fixed relative to the electrical field generating means. Hence, even a change in the electrical field cannot cause a movement of the first molecular element. The first molecular element rather acts as a base for movement of the second molecular element. The second molecular element is therefore the only part which can be moved in response to the electrical field.

At least the second molecular element is electrically charged. The second molecular element can be an electrically charged molecule, particularly a biomolecule, or can be artificially electrically charged. In particular, an electrically charged functional group can be added to a molecular structure in order to create the electrically charged second molecular element. Due to the electrical charge, the second molecular element aligns to said electrical field. In case the electrical field is changed in orientation, the orientation of the second molecular element is also changed. This means that the control part can control fine adjustment of the second molecular element with respect to the first molecular element. The second molecular element can be transferred to and held in any orientation with respect to the first molecular element that is not prevented by the linking element. Additionally, a continuous movement of the second molecular element can be realized.

In summary, the movement part sets up the kinematics of the molecular machine, while the control part powers and controls any movement of said kinematics via electric actuation. Electric actuation solves several technical challenges that are currently faced by molecular nanomachines. In particular, electric actuation allows controlling molecular switches and mechanisms faster, with higher precision, and with less complex instrumentation compared to conventional methods. Moreover, the invention offers the solution to a central challenge of nanomanipulation (the "fat fingers" problem) since the externally controlled nanomanipulators are of the same small length scale as the manipulated nanoscale objects and molecules.

In a preferred embodiment, the control part comprises a fluidic channel. The movement part is provided in the fluidic channel. The control part further comprises an electrical device including electrodes. The electrodes are connected to the fluidic channel. In this way, the electrical field as described above can be created. The electrical device comprises a voltage source and electrical wiring to apply the voltage to the electrodes. Preferably, the electrical device comprises two electrodes for generating the electrical field. Alternatively, the voltage source can be a three-phase voltage source such that the electrical device has three electrodes. This allows providing a rotating electrical field such that the second molecular element can be rotated in a simple way.

Preferably, the control part comprises at least two electrical devices and fluidic channels with different orientations. In this way, two independent overlaying electrical fields can be created. The fluidic channels are arranged to intersect at an intersection area and the movement part is placed at the intersection area. Therefore, a two-dimensional movement of the second molecular element can be controlled. Alternatively, the above described three-phase voltage and the three electrodes might be used for two-dimensional movement control.

Favorably, the first molecular element is fixed to the fluidic channel. This means that the first molecular element can not move in response to the electrical field. The molecular element rather is a fixed base for the movement of the second molecular element. In this way, it is ensured that only the second molecular element can be moved. Further the fixation of the first molecular element allows very fine adjustment of the second molecular element, which aligns to the electrical field while the first molecular element does not move and/or align to the electrical field.

Providing the electrical device including electrodes might cause unwanted electrochemical effects. Particularly in case the movement part is employed for synthesis purposes, electrochemical effects to the synthesized products should be avoided. Therefore, the electrical device preferably includes an isolating element for isolating the electrodes from the movement part. The isolating elements particularly comprise membranes and/or gels and/or salt bridges. This means that only selected molecules can pass through the isolating elements such that the electrodes are separated from the movement part. However, the electrical field generated by the electrical devices is not influenced or at least not significantly influenced by the isolating element. Therefore, the control of movement of the second molecular element is not affected. The isolating element preferably also reduces the volume provided for reactions. This particularly allows holding the components of a desired reaction close to the movement part such that the components can manipulated and/or moved by the molecular machine.

The linking element is favorably part of the first molecular element or the second molecular element. Particularly, the first molecular element and/or the second molecular element might comprise a functional group which is adapted to link the first molecular element and the second molecular element. In this way, the manufacturing process of the molecular machine is simplified. Additionally, the linking element can be part of at least one of two or more mechanically interlocked molecules. This is particularly preferred in a case in which the movement part comprises rotaxanes. In such structures, the first molecular element comprises a linear part and the linking element and the second molecular element comprises a ring structure. The ring structure can rotate about the linear part and the linking element avoids the ring structure slipping off the linear part.

The first molecular element and/or the second molecular element and/or the linking element preferably are biomolecules. The biomolecules are particularly electrically charged. In a further preferred embodiment, the first molecular element and/or the second molecular element and/or the linking element are made of DNA (deoxyribonucleic acid), preferably DNA-origami, and/or RNA (ribonucleic acid) and/or protein and/or artificial charged supramolecular structures.

In a preferred embodiment, the first molecular element is a platform and the second molecular element is a positioning arm. The positioning arm is fixed to the platform via the linking element. The linking element constrains all relative movement of the first molecular element and the second molecular element except of a rotation of the second molecular element within a plane parallel to the first molecular element. Therefore, the positioning arm is preferably moved by aligning to two overlaid electrical fields. This allows adjusting the positioning arm in relation to the platform. Particularly, a full rotation of the positioning arm is possible, wherein the positioning arm can be stopped and hold in any position. Further, high forces are generated which allow manipulation of further molecules.

The first molecular element and/or the second molecular element are addressable. This means, that functional groups can be provided on the first molecular element and the second molecular element. Preferably, both, the first molecular element and the second molecular element can be addressed. Therefore, the movement part can be adapted to specific needs. This allows employing the molecular machine in various environments and/or for various purposes.

Preferably, fluctuations of the first molecular element and/or the second molecular element due to diffusion are within a tolerance of at most 10 nm, preferably at most 1 nm, particularly preferable at most 0.5 nm. Hence, a fine adjustment of the second molecular element is facilitated.

Preferably, any dimension of the first molecular element and the second molecular element is less than 1000 nm. Particularly, the above described platform is preferably made of square shape with a length of 50 nm. The positioning arm is preferably shorter than said length. Particularly, the positioning arm is adequately addressable within the overlap with the platform. Such dimensions allow manipulation of molecules with the molecular machine. Therefore, the molecular machine is a molecular nanomechanism.

A specific embodiment will be described together with the attached drawings. In the drawings,
- Fig. 1: is a schematic view of a moving part of a molecular machine according to an embodiment of the invention,
- Fig. 2: is a schematic view of a first alternative of a control part of the molecular machine according to the embodiment of the invention,
- Fig. 3: is a schematic view of a second alternative of a control part of the molecular machine according to the embodiment of the invention,
- Fig. 4: is a schematic view of the moving part of the molecular machine according to the embodiment of the invention, which is modified for proof of functionality,
- Fig. 5: is a schematic view of montage of single images from a microscope video showing the movement of the molecular machine according to the embodiment of the invention, and
- Fig. 6: is a schematic view of a diagram showing movement of the molecular machine according to the embodiment of the invention.

### Description of the embodiment

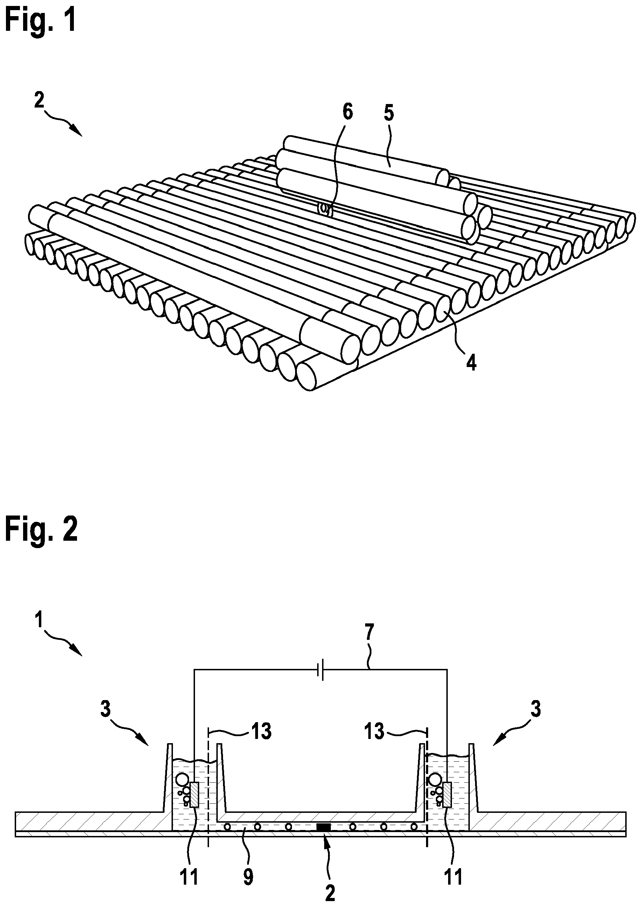

Figure 1 is a schematic view of a movement part 2 of a molecular machine 1 according to an embodiment of the invention. In the embodiment, the movement part 2 comprises a platform 4, which corresponds to the first molecular element. On the platform 4, a position arm 5 is mounted, which corresponds to the second molecular element. A linking element 6 constrains the relative movement between platform 4 and positioning arm 5 such that the only possible relative movement is a rotation of the positioning arm 5 in a plane parallel to the platform 4.

Platform 4 and positioning arm 5 are constructed with the DNA origami method. The DNA origami method is well-known in the art and is for example described in prior art references 17 to 18. The square platform 4 consists of two layers of DNA double helices. In all figures, each double helix is represented by a cylinder. The positioning arm 5 is a six helix bundle. The linking element 7 comprises two DNA strands and connects the positioning arm 5 to the platform 4. Transmission electron microscopy micrographs can show the structure and quality of these objects.

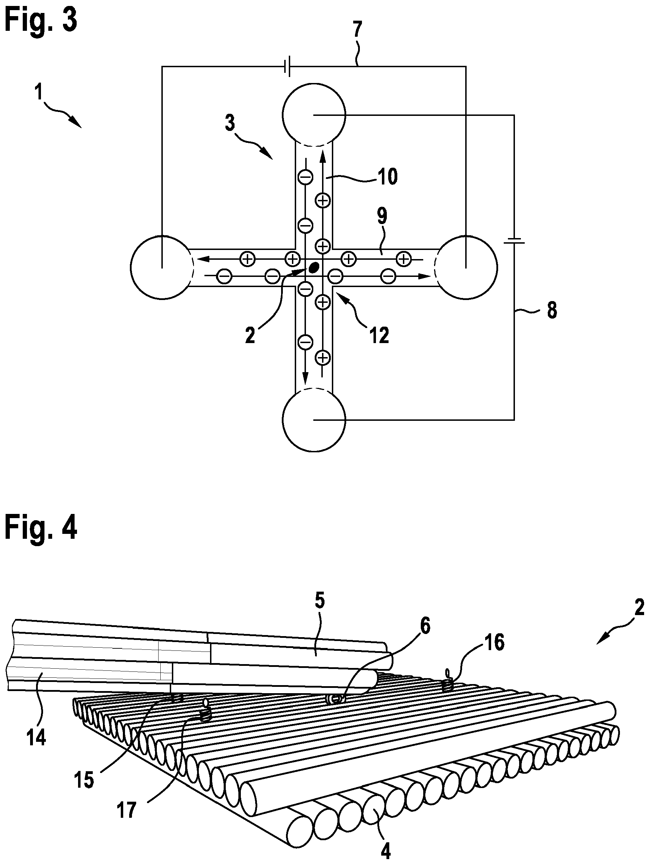

The platform 4 and the positioning arm 5 can be built from different molecules e.g. RNA, proteins, artificial charged supramolecular structures. The positioning arm 5 might be elongated by coupling to further structures. An example for such elongation is described with respect to figure 4.

DNA molecules and thus also DNA origami structures are heavily charged biomolecules that can be electrophoretically manipulated. This fact can be exploited to achieve control and movement of molecular mechanisms.

Electric fields can be created in a simple electrophoretic or micro-electrophoretic setup. In the embodiment, a control part 3 is employed, which is shown in figures 2 and 3. Figure 2 is a schematic view of the molecular machine 1 including a control part 3 according to a first alternative, while figure 3 is a schematic view of the molecular machine including a control part 3 according to a second alternative.

As shown in figure 2, a fluidic channel 9 is provided which is contacted by platinum electrodes 11 of an electrical device 7. The movement part 2 is placed in the center of this fluidic channel 7, preferably as far away from the electrodes as possible. Isolating elements 13 might be provided in order to isolate the electrodes 13 from the movement part 2. The Isolating elements 13 preferably comprise membranes, gels or salt bridges. The isolating elements 13 do not allow transfer of selected molecules such that the electrodes are separated form the movement part 2 such that no unwanted traveling of elements from the movement part 2 to the electrodes 11 can take place. This avoids unwanted electrochemical influences of the electrodes 11.

Electric control is achieved by applying voltages to the electrodes. For that purpose, low control voltages as output channels of a DAQ board (data acquisition board) are amplified to adequate voltages by an operational amplifier. In the embodiments, the electrical device 7 applies voltages of 200 V.

Two-dimensional movement of the positioning arm 5 can be realized with a crossed channel geometry. This is shown in figure 3. The control part 3 comprises a first electrical device 7 and a second electrical device 8, both are identical to the above described electrical device of figure 2. The first electrical device 7 is connected to a first fluidic channel 9 and the second electrical device 9 is connected to a second fluidic channel 10, wherein the first fluidic channel 9 and second fluidic channel 10 are both identical as the above described fluidic channel of figure 2. The first fluidic channel 9 and the second fluidic channel 10 intersect at an intersection area 12 and are orientated perpendicular to each other. The movement part 2 is placed in the intersection area 12. Due to such a design, the electrical fields of the first electrical device 7 and the second electrical device 8 are superposed. Superposition of electrical fields in the fluidic channels 9, 10 allows to adjust the positioning arm 5 in arbitrary angles or to rotate it in circles relative to the platform 4.

In order to ensure that only the positioning arm 5 rotates while the platform 4 remains still, the platform 4 is fixed to at least one of the fluidic channels 9, 10 of the control part 3. In this way, the platform is fixed relative to the control part 3 which means that no movement of the platform 4 is possible in response to the electrical field.

In an alternative setup, lithographically designed microelectrodes can be used, which require much smaller voltages for manipulation. In principle, this enables the miniaturization of the whole setup and the integration e.g. on a USB-Stick.

The electrically charged positioning arm 5 aligns to the electrical fields generated via the first electrical device 7 and the second electrical device 8. In this way, an exact positioning of the positioning arm 5 can be reached. Particularly, the positioning arm 5 can be moved with a tolerance of at most 1 nm.

### Proof of Functionality

Evidence for the electro-controlled movement of positioning arms 5 can be provided in several ways.

Figure 4 is a sketch of the DNA platform 4 with the positioning arm 5 that is modified with a blue donor dye 15. The platform 4 was labeled at two positions with a green first acceptor dye 16 and red second acceptor dye 17, respectively. The positioning arm 5 movement can be proven via single molecule FRET (fluorescence resonance energy transfer). Further, for better electric coupling (via the DNA-structures charge) and to enable direct optical observation an additional lever/pointer structure 14 is provided to extend the positioning arm 5 to a length of several 100 nm (in this example ∼400nm).

The movement of the positioning arm can be characterized by means of single molecule fluorescence resonance energy transfer (smFRET), which demonstrates the system's positioning precision on the nanometer scale. As the blue donor dye 15, Alexa Fluor 488 is employed, as the green first acceptor dye 16, ATTO 565 is employed, and as the red second acceptor dye 17, ATTO 647N is employed. The blue donor dye 15 can excite the first acceptor dye 16 and second acceptor dye 17 via FRET if donor and acceptor are in closer proximity than the Foerster radius (∼ 6 nm).

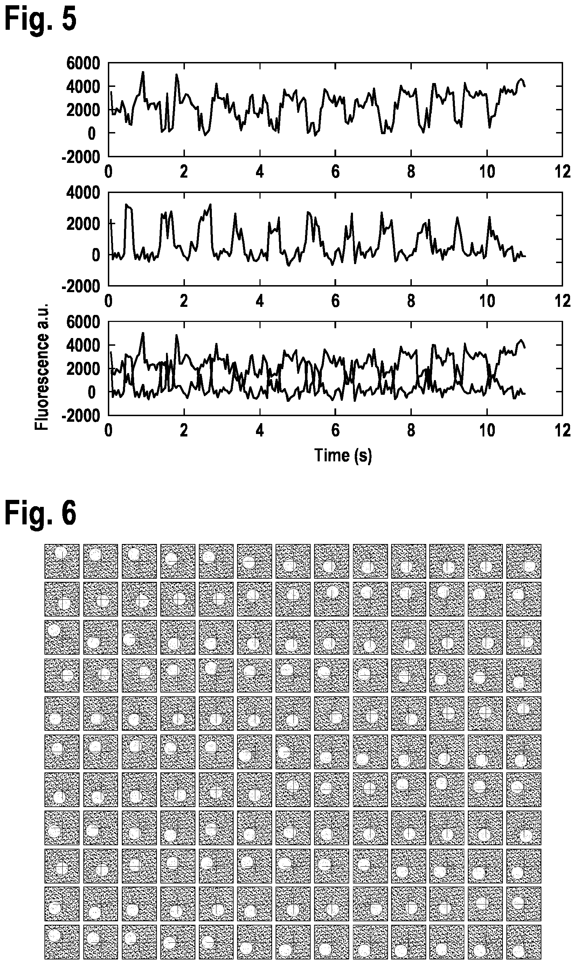

Figure 5 illustrates a single molecule FRET traces corresponding to the sketch in figure 4. The rotation of the positioning arm 5 is externally driven at 1 Hz while the donor dye 15 on the positioning arm 5 is being excited. This results in an alternating emission of green by the first acceptor dye 16, as shown in the top diagram of figure 5, and red by the second acceptor dye 17, as shown in the middle diagram of figure 5. An overlay of both traces, which is shown in the bottom diagram of figure 5, clearly shows the periodic alternating excitation.

As shown in figure 6, the invention can prove a periodic movement of the positioning arm 5 from one position of the platform 4 to the other. This experiment also demonstrates the potential of the invention to transport and position molecules on the platform 4, which is of great importance for a wide range of applications.

An alternative way of proof of movement of the positioning arm 5 is shown in Figure 6. Figure 6 shows a montage of single images from a microscope video, which shows the electrically driven rotation of the positioning arm (exposure per frame: 50 ms, Scale bar: 500 nm). Fluorescent dyes are fixed to the tip of positioning arm 5. The origami platform 4 is located in the image center and is indicated by a cross. The particle rotates in an external field. For figure 6, fluorescent dyes have been placed on the tip of the extension of the positioning arm 5, i.e. on the additional lever/pointer structure 14. The positioning arm 5 is rotated in circles with a frequency of 1 Hz. As shown on the image series, the particle at the tip of the positioning arm is performing the desired rotational movement. This also shows the controlled movement of a nanoscale object on a length scale of 1 µm.

### Industrial Application Perspectives

The key capability to position molecules precisely, fast and electrically controlled as well as the possibility to locally exert directed forces trough molecular mechanisms on the nanoscale enables a wide variety of application opportunities in nanotechnology. Below, three possible areas of application will be briefly discussed.

### Single Molecule Sensing and Force Spectroscopy

Highly specific molecular interactions are responsible for a wide range of biological processes and are also the mechanism of action of pharmacological substances. For this reason, biological research has been focused on the precise biochemical and biophysical characterization of these interactions for quite some time. In biophysical research, the strength of interactions is often analyzed in single molecule experiments. Here special instruments are used to exert forces on the binding partners. This includes experiments with atomic force microscopes, optic tweezers and magnetic tweezers.

The movement part 2 of platform 4 and positioning arm 5 according to the described embodiment of the invention make it possible to apply forces to molecules in situ. That is to say, in case of the invention, the force applying lever itself is a molecular structure. Contrary to the other methods mentioned, it is relatively simple to conjugate the molecules and binding partners that are to be characterized to the platform 4 and the positioning arm 5. The experimental setup used to create electric forces is much simpler. This enables highly parallelizable execution of force measurements of molecular interaction partners since a vast number of measuring platforms 4 can be actuated at the same time. The sensor principle can also be used for screening of molecule libraries by "barcoding" (cf. prior art reference 19) of single platforms 4.

### DNA Templated Synthesis

In the past 15 years, DNA templated synthesis was established as a novel method to increase the efficiency of chemical reactions and for the sequence based production of molecule libraries. This approach exploits the highly increased local concentration of molecules that were conjugated to a DNA strand and are thereby colocalized by sequence specific base pairing on the template (cf. prior art references 20 and 21).

This principle can be transferred to reactions with electrically driven molecular mechanisms. Electrically addressable moving molecular mechanisms can bring molecules into close proximity to induce their reaction. In this way for example, the same reaction can be repeated depending on an external clocking signal or sequential reactions can be performed according to a programmable protocol. Contrary to existing "proof of principle" experiments, the possibility of repeated and highly parallelized performance of such reactions enable the production of technologically relevant amounts of substances. The invention, i.e. the development of the electrical switchable molecular machine 1, is therefore an enabling technology for the realization of genuine molecular robotic systems and molecular assembly lines.

### Photonics/Plasmonics

The molecular actuators according to the invention can readily be modified with inorganic particles like e.g. metal or semiconductor particles. For instance, a change in position of the molecular mechanisms can vary the particles' orientation with respect to a polarized external field. Accordingly, the occurrence of plasmonic effects (e.g. field enhancement, energy transfer, heating effects) can be switched via electric control.

### Summary

With the described invention, nanoscale objects or molecules can be controllably moved and positioned. As an embodiment and for the demonstration of the general working principle a molecular positioning arm 5 from DNA molecules was explained. The nanoscale molecular positioning arm 5 is fixated on a specifically addressable platform 4 with a flexible joint that allows rotation around the pivot point. The positioning arm 5 movement can be precisely controlled by external electric fields. The positioning arm 5 can transport molecules, control chemical processes and exert forces on other molecules "in situ". The method exploits the intrinsic electric charge of biomolecules and can be generally applied to synthetic as well as naturally occurring biomolecular mechanisms.

### List of reference signs

- 1: molecular machine
- 2: movement part
- 3: control part
- 4: platform (first molecular element)
- 5: positioning arm(second molecular element)
- 6: linking element
- 7: first electrical device
- 8: second electrical device
- 9: first fluidic channel
- 10: second fluidic channel
- 11: electrode
- 12: intersection area
- 13: isolating element
- 14: additional lever/pointer structure
- 15: donor dye
- 16: first acceptor dye
- 17: second acceptor dye

## Claims

1. Molecular machine (1) comprising
• a movement part (2) including
∘ a first molecular element (4),
∘ a second molecular element (5), and
∘ a linking element (6) for constraining a relative movement of the first molecular element (4) and the second molecular element (5), and
• a control part (3) configured to generate an electrical field around the movement part (2),
• wherein the first molecular element (4) is fixed relative to the control part (3),
• wherein the second molecular element (5) is movable relative to the first molecular element (4) in at least one degree of freedom, and
• wherein the second molecular element (5) is electrically charged such that the second molecular element (5) aligns to said electrical field.

2. Molecular machine (1) according to claim 1, **characterized in that** the control part (3) comprises a fluidic channel (9, 10), in which the movement part (2) is provided, wherein the control part (3) has an electrical device (7, 8) including electrodes (11), which are connected to the fluidic channel (9, 10), for creating the electrical field.

3. Molecular machine (1) according to claim 2, **characterized in that** the control part (3) comprises at least two electrical devices (7, 8) and fluidic channels (9, 10) with different orientations in order to create at least two independent overlaying electrical fields, wherein the fluidic channels (9, 10) are arranged to intersect at an intersection area (12) and the movement part (2) is placed at the intersection area (12).

4. Molecular machine (1) according to claim 2 or 3, **characterized in that** the first molecular element (4) is fixed to the fluidic channel (9, 10).

5. Molecular machine (1) according to any one of the claims 2 to 4, **characterized in that** the electrical device (7, 8) includes an isolating element (13) for isolating the electrodes (11) from the movement part (2).

6. Molecular machine (1) according to any one of the previous claims, **characterized in that** the linking element (6) is part of the first molecular element (4) or the second molecular element (5).

7. Molecular machine (1) according to any one of the previous claims, **characterized in that** the first molecular element (4) and/or the second molecular element (5) and/or the linking element (6) are biomolecules, preferably made of DNA, preferably DNA-origami, and/or RNA and/or protein and/or artificial charged supramolecular structures.

8. Molecular machine (1) according to any one of the previous claims, **characterized in that** the first molecular element (4) is a platform and the second molecular element (5) is a positioning arm, wherein the linking element (6) constrains all relative movement of the first molecular element (4) and the second molecular element (5) except of a rotation of the second molecular element (5) within a plane parallel to the first molecular element (4).

9. Molecular machine (1) according to any one of the previous claims, **characterized in that** fluctuations of the first molecular element (4) and/or the second molecular element (5) due to diffusion are within a tolerance of at most 10 nm, preferably at most 1 nm, particularly preferred at most 0.5 nm.

10. Molecular machine (1) according to any one of the previous claims, **characterized in that** any dimension of the first molecular element (4) and the second molecular element (5) is less than 1000 nm.
